(19) Europäisches Patentamt
European Patent Office
Office européen des brevets

(11) **EP 2 528 630 B1**

(12) **EUROPÄISCHE PATENTSCHRIFT**

(45) Veröffentlichungstag und Bekanntmachung des Hinweises auf die Patenterteilung:
**30.03.2016 Patentblatt 2016/13**

(21) Anmeldenummer: **11701493.6**

(22) Anmeldetag: **20.01.2011**

(51) Int Cl.:
*A61L 15/18* (2006.01)       *A61L 15/46* (2006.01)

(86) Internationale Anmeldenummer:
**PCT/EP2011/050713**

(87) Internationale Veröffentlichungsnummer:
**WO 2011/092098 (04.08.2011 Gazette 2011/31)**

(54) **GERUCHSINHIBIERENDE WASSERABSORBIERENDE VERBUNDSTOFFE**

ODOR-INHIBITING, WATER-ABSORBING COMPOSITE MATERIALS

MATIÈRES COMPOSITES ABSORBANT L'EAU ET INHIBANT LES ODEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.01.2010 EP 10151798**

(43) Veröffentlichungstag der Anmeldung:
**05.12.2012 Patentblatt 2012/49**

(73) Patentinhaber: **BASF SE**
**67056 Ludwigshafen (DE)**

(72) Erfinder:
• **BRAIG, Volker**
**69469 Weinheim-Lützelsachsen (DE)**
• **DANIEL, Thomas**
**67165 Waldsee (DE)**

(56) Entgegenhaltungen:
**WO-A2-2010/096595    GB-A- 627 512
US-A- 4 363 322    US-B1- 6 649 805**

**Beschreibung**

Die vorliegende Erfindung betrifft ein Verfahren zur Herstellung geruchsinhibierender wasserabsorbierender Verbundstoffe, enthaltend wasserabsorbierende Polymerpartikel und Fasern, wobei die Fasern mit mindestens einem Metallperoxid, Metallhyperoxid oder Metallozonid vorgemischt werden.

Die Herstellung von Hygieneartikeln wird in der Monographie "Modern Superabsorbent Polymer Technology", F.L. Buchholz und A.T. Graham, Wiley-VCH, 1998, Seiten 252 bis 258, beschrieben.

Hygieneartikel bestehen üblicherweise aus einer oberen flüssigkeitsdurchlässigen Abdeckung, einer unteren flüssigkeitsundurchlässigen Schicht und einem zwischen der Abdeckung und der Schicht befindlichen wasserabsorbierenden Verbundstoff. Der Verbundstoff besteht dabei aus wasserabsorbierenden Polymeren und Fasern. Weitere Schichten sind beispielsweise Aufnahme- und Verteilschichten und/oder Tissueschichten.

Die Herstellung wasserabsorbierender Polymerpartikel wird ebenfalls in der Monographie "Modern Superabsorbent Polymer Technology", F.L. Buchholz und A.T. Graham, Wiley-VCH, 1998, Seiten 71 bis 103, beschrieben. Die wasserabsorbierenden Polymerpartikel werden auch als Superabsorber bezeichnet.

Die Eigenschaften der wasserabsorbierenden Polymerpartikel können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsweiterleitung im gequollenen Gelbett (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen des hydrophilen Polymeren kovalente Bindungen bilden können.

GB 627,512 offenbart die Verwendung von Zinkperoxid zur Geruchsinhibierung in Hygieneartikeln.

GB 2 377 890 beschreibt Oxidationsmittel als geruchsinhibierende Zusatzstoffe in was serabsorbierenden Zusammensetzungen.

JP 2001/39802 lehrt die Verwendung von Natriumperkarbonat und Natriumperborat als antimikrobielle Zusätze für wasserabsorbierende Zusammensetzungen.

JP 2001/115042 offenbart wasserabsorbierende Zusammensetzungen, enthaltend wasserabsorbierende Polymerpartikel, anorganische Peroxide und Ethylendiamintetraessigsäure.

Aufgabe der vorliegenden Erfindung war die Bereitstellung verbesserter geruchsinhibierender wasserabsorbierender Verbundstoffe.

Gelöst wurde die Aufgabe durch Verfahren zur Herstellung geruchsinhibierender wasserabsorbierender Verbundstoffe, enthaltend wasserabsorbierende Polymerpartikel und Fasern, dadurch gekennzeichnet, dass die Fasern mit mindestens einem Metallperoxid. Metallhyperoxid oder Metallozonid vorgemischt werden.

Das Metallperoxid ist vorzugsweise das Peroxid eines Metalls der 1. Hauptgruppe, der 2. Hauptgruppe und/oder der 2. Nebengruppe des Periodensystems der Elemente, besonders bevorzugt das Peroxid eines Metalls der 2. Nebengruppe des Periodensystems der Elemente, ganz besonders bevorzugt Zinkperoxid.

Geeignete Metallperoxide sind beispielsweise Lithiumperoxid, Strontiumperoxid, Bariumperoxid, Natriumperoxid, Magnesiumperoxid, Kalziumperoxid, Kaliumhyperoxid und Zinkperoxid. Die Metallperoxide liegen üblicherweise als Gemische mit ihren Oxiden vor, beispielsweise Zinkperoxid mit Zinkoxid.

Zur Verminderung der Korrosion sollten die Metallperoxide, Metallhyperoxide bzw. Metallozonide möglichst wenig Halogenid wir Clorid enthalten, vorzugsweise weniger als 500 ppm, besonders bevorzugt weniger als 200 ppm, ganz besonders bevorzugt weniger als 10 ppm.

Der Verbundstoff enthält vorzugsweise 0,001 bis 5 Gew.-%, bevorzugt von 0,01 bis 3 Gew.-%, besonders bevorzugt von 0,1 bis 1,5 Gew.-%, ganz besonders bevorzugt von 0,2 bis 0,8 Gew.-%, des Metallperoxids, Metallhyperoxids oder Metallozonids.

US 6,649,805 offenbart die Verwendung von Peroxisäuren in Hygieneartikeln zur Geruchsinhibierung.

WO 2010/096595 beschreibt Superabsorber, die mit einer Lösung aus Wasserstoffperoxid oder Wasserstoffperoxid und Metallsalzen behandelt werden sowie die Verwendung dieser Superabsorber in Hygieneartikeln.

Der Verbundstoff enthält vorzugsweise von 10 bis 90 Gew.-%, besonders bevorzugt von 30 bis 80 Gew.-%, ganz besonders bevorzugt von 50 bis 70 Gew.-% Fasern. Die bevorzugten Fasern sind Zellulosefasern.

Der vorliegenden Erfindung liegt die Erkenntnis zugrunde, dass die geruchsinhibierende Wirkung von Metallperoxiden, Metallhyperoxiden und Metallozoniden, insbesondere Zinkperoxid, durch Vormischen mit den Fasern deutlich gesteigert werden kann.

Die erfindungsgemäßen Verbundstoffe enthalten vorzugsweise weniger als 50 ppm, besonders bevorzugt weniger als 10 ppm, ganz besonders bevorzugt weniger als 5 ppm, Schwermetallionen. Schwermetallionen, insbesondere Eisenionen, führen zur katalytischen Zersetzung der Metallperoxide, Metallhyperoxide und Metallozonide und senken somit die Lagerstabilität der Verbundstoffe.

Im Folgenden wird die Herstellung der wasserabsorbierenden Polymerpartikel und der Verbundstoffe näher erläutert.

Die wasserabsorbierenden Polymerpartikel werden beispielsweise durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS). Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1,

EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach

ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ (AUL0.3psi) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Zellulose, wie Methylzellulose oder Hydroxyethylzellulose, Gelatine, Polyglykole oder Polyacrylsäuren, vorzugsweise Stärke, Stärkederivate und modifizierte Zellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, für "saure" Polymergele besonders bevorzugt von 30 bis 60 mol-%, ganz besonders bevorzugt von 35 bis 55 mol-%, für "neutrale" Polymergele besonders bevorzugt von 65 bis 80 mol-%, ganz besonders bevorzugt von 70 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze wie das Salz des Triethanolamins verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Flüssigkeitsweiterleitung (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 $C_1$ Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform werden vor, während oder nach der Oberflächennachvernetzung zusätzlich zu den Oberflächennachvernetzern polyvalente Kationen auf die Partikeloberfläche aufgebracht.

Die einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, vierwertige Kationen, wie die Kationen von Titan und Zirkonium. Als Gegenion sind beispielsweise Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Flüssigkeitsweiterleitung (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verpackungsneigung der Polymerpartikel sind beispielsweise pyrogene Kieselsäure, wie Aerosil® 200, und Tenside, wie Span® 20.

Die wasserabsorbierenden Polymerpartikel weisen einen Feuchtegehalt von vorzugsweise 1 bis 15 Gew.-%, besonders bevorzugt 2 bis 10 Gew.-%, ganz besonders bevorzugt 3 bis 5 Gew.-%, auf, wobei der Feuchtegehalt gemäß der von der EDANA empfohlenen Testmethode Nr. WSP 230.2-05 "Moisture Content" bestimmt wird.

Die wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Centrifuge Retention Capacity" bestimmt.

Die wasserabsorbierenden Polymerpartikel weisen eine Absorption unter einem Druck von 49,2 $g/cm^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 $g/cm^2$ der wasserabsorbierenden Polymerpartikel beträgt üblicherweise weniger als 35 g/g. Die Absorption unter einem Druck von 49,2 $g/cm^2$ wird analog der von der EDANA empfohlenen Testmethode Nr. WSP 242.2-05 "Absorption under Pressure" bestimmt, wobei statt eines Drucks von 21,0 $g/cm^2$ ein Druck von 49,2 $g/cm^2$ eingestellt wird.

Der wasserabsorbierende Verbundstoff enthält neben den wasserabsorbierenden Polymerpartikeln mindestens ein, vorzugsweise hydrophiles, Fasermaterial. Unter hydrophil ist zu verstehen, dass sich wässrige Flüssigkeiten schnell über die Faser verteilen. Für gewöhnlich ist das Fasermaterial Zellulose, modifizierte Zellulose, Rayon, Polyester wie Polyethylenterephthalat. Besonders bevorzugt werden Zellulosefasern wie Zellstoff. Die Fasern haben in der Regel einen Durchmesser von 1 bis 200 μm, bevorzugt 10 bis 100 μm. Darüber hinaus haben die Fasern eine Mindestlänge von 1 mm.

Einen detaillierten Überblick über Beispiele von Fasern, die in der vorliegenden Erfindung eingesetzt werden können, gibt die Patentanmeldung WO 95/26209 A1, Seite 28, Zeile 9 bis Seite 36, Zeile 8. Besagte Passage ist somit Bestandteil dieser Erfindung.

Beispiele für Zellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Zellulosefasern wie Baumwolle, Flachs, Seide, Wolle, Jute, Ethylzellulose und Zelluloseazetat Anwendung.

Geeignete synthetische Fasern werden hergestellt aus Polyvinylchlorid, Polyvinylfluorid, Polytetrafluorethylen, Polyvinylidenchlorid, Polyacrylverbindungen wie ORLON®, Polyvinylazetat, Polyethylvinylazetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DACRON® oder KODEL®), Copolyester, Polyvinylazetat, Polyethylvinylazetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Der Faserquerschnitt kann rund oder eckig sein, sowie eine andere Form haben beispielsweise Schmetterlingsartig.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt von 75 bis 175°C aufweisen. Bei diesen Temperaturen ist noch keine Schädigung der Zellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9.000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10.000 Meter). Die Form ist nicht besonders eingeschränkt und Beispiele schließen gewebeartige, schmale zylinderartige, geschnitten-/spaltgarnartige, stapelfaserartige und endlosfaserartige ein.

Geeignete hydrophile Fasern sind beispielsweise Zellulosefasern, modifizierte Zellulosefasern, Rayon, Polyesterfasern wie beispielsweise Polyethylenterephthalat (DACRON®), und hydrophiles Nylon (HYDROFIL®). Geeignete hydrophile Fasern können auch erhalten werden durch Hydrophilierung hydrophober Fasern, wie beispielsweise die Behandlung thermoplastischer Fasern, erhalten aus Polyolefinen (beispielsweise Polyethylen oder Polypropylen, Polyamide, Polystyrole, Polyurethane usw.) mit Tensiden oder Silica. Aus Kostengründen und aus Gründen der Verfügbarkeit werden jedoch Zellulosefasern bevorzugt.

Gemäß dem erfindungsgemäßen Verfahren werden die Fasern mit dem mindestens einen Metallperoxid, Metallhyperoxid oder Metallozonid vorgemischt. Die Art des Mischens unterliegt keiner Beschränkung. Es können beispielsweise Pulvermischungen aus Fasern und Metallperoxid, Metallhyperoxid oder Metallozonid hergestellt werden. Es ist aber auch möglich das Metallperoxid, Metallhyperoxid oder Metallozonid als Lösung oder Suspension aufzusprühen.

Bei der Herstellung von Pulvermischungen aus Fasern und mindestens einem Metallperoxid, Metallhyperoxid oder Metallozonid werden vorteilhaft Entstaubungsmittel eingesetzt. Geeignete Entstaubungsmittel sind Polyglyzerine, Polyethylenglykole, Polyproplenglykole, statistische oder Block-Copolymere von Ethylenoxid und Propylenoxid. Weitere zu diesem Zweck geeignete Entstaubungsmittel sind die Polyethoxylate oder Polypropoxylate von Polyhydroxyverbindungen, wie Glyzerin, Sorbitol, Trimethylolpropan, Trimethylolethan und Pentaerythrit. Beispiele hierfür sind n-fach ethoxyliertes Trimethylolpropan oder Glyzerin, wobei n eine ganze Zahl zwischen 1 und 100 darstellt. Weitere Beispiele sind Block-Copolymere, wie insgesamt n-fach ethoxyliertes und dann m-fach propoxyliertes Trimethylolpropan oder Glyzerin, wobei n eine ganze Zahl zwischen 1 und 40 und m eine ganze Zahl zwischen 1 und 40 darstellt. Die Reihenfolge der Blöcke kann auch umgekehrt sein. Die Entstaubungsmittel können auch mit Wasser verdünnt werden.

Die wasserabsorbierenden Polymerpartikel werden in das beschriebene Fasermaterial eingebettet. Dies kann auf vielfältige Weise geschehen, indem man beispielsweise mit den wasserabsorbierenden Polymerpartikeln und den Fasern zusammen eine Absorptionsschicht in Form einer Matrix aufbaut, oder durch Einlagerung wasserabsorbierender Polymerpartikel in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Zellulosefaser oder einem Gemisch aus synthetischer Faser und Zellulosefaser bestehen, wobei das Mischungsverhältnis von (100 bis 0) synthetische Faser : (0 bis 100) Zellulosefaser variieren kann. Die eingesetzten Zellulosefasern können zur Erhöhung der Formbeständigkeit zusätzlich chemisch versteift sein.

Die chemische Versteifung von Zellulosefasern kann auf unterschiedlichen Wegen erreicht werden. Zum einen kann eine Faserversteifung erreicht werden durch Zusatz geeigneter Überzüge oder Beschichtungen zum Fasermaterial. Derartige Zusätze schließen beispielsweise Polyamid-Epichlorhydrin-Überzüge (Kymene®557 H, Hercules, Inc., US), Polyacrylamid- Überzüge (beschrieben in US 3,556,932 oder als Handelsprodukt der Marke Parez® 631 NC, American Cyanamid Co., US), Melamin-Formaldehyd-Überzüge und Polyethylenimin-Überzüge mit ein.

Die chemische Versteifung von Zellulosefasern kann auch durch chemische Reaktion erfolgen. So kann beispielsweise die Zugabe von geeigneten Vernetzer eine Vernetzung bewirken, die innerhalb der Faser stattfindet. Geeignete Vernetzer sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielsweise Glutaraldehyd, Glyoxal, Glyoxylsäure, Formaldehyd und Zitronensäure. Diese Substanzen reagieren mit mindestens zwei HydroxylGruppen innerhalb einer einzelnen Zellulosekette oder zwischen zwei benachbarten Zelluloseketten innerhalb einer einzelnen Zellulosefaser. Durch die Vernetzung erfolgt eine Versteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Zellulosefasern sind bekannt und in WO 91/11162 A1, US 3,224,926, US 3,440,135, US 3,932,209, US 4,035,147, US 4,822,453, US 4,888,093, US 4,898,642 und US 5,137,537 beschrieben. Die chemische Vernetzung bewirkt eine Versteifung des Fasermaterials, was sich letztendlich in einer verbesserten Formbeständigkeit des gesamten Verbundstoffs widerspiegelt. Die einzelnen Schichten werden durch dem Fachmann bekannte Methoden, wie beispielsweise Verschmelzen durch Wärmebehandlung, Zugabe von Schmelzklebern, Latexbindern usw. miteinander verbunden.

Beispiele für Verfahren, mit denen man einen wasserabsorbierenden Verbundstoff erhält, die beispielsweise aus einem Trägermaterial bestehen, an den ein- oder beidseitig wasserabsorbierende Polymerpartikel fixiert sind, sind bekannt und von der Erfindung mit eingeschlossen, jedoch nicht limitiert darauf.

Beispiele für Verfahren, mit denen man einen wasserabsorbierenden Verbundstoff erhält, die beispielsweise aus in ein Fasermaterial-Gemisch aus synthetischen Fasern (a) und Zellulosefasern (b) eingebetteten wasserabsorbierende Polymerpartikel (c) besteht, wobei das Mischungsverhältnis von (100 bis 0) synthetische Faser: (0 bis 100) Zellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Der entsprechend erzeugte einen wasserabsorbierenden Verbundstoff kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunden bis 3 Minuten, bevorzugt 1 Sekunde bis 1 Minute.

Der entsprechend hergestellte wasserabsorbierende Verbundstoff kann optional einer Verpressung unterworfen werden, vorzugsweise mit einer Rollenpresse. Die Rollen können beheizt sein. Die Spaltweite gibt den Pressgrad vor. Die Spaltbreite beträgt üblicherweise von 1 bis 100% der ursprünglichen Dicke des Materials.

Der wasserabsorbierende Verbundstoff wird im Allgemeinen mit einer für Flüssigkeit durchlässigen Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in der WO 95/26209 A1.

Die vorliegende Erfindung betrifft ferner die Verwendung der oben genannten wasserabsorbierenden Verbundstoffe in Hygieneartikeln. Beispielsweise kann der Hygieneartikel wie folgt aufgebaut sein:

(A) eine obere flüssigkeitsdurchlässige Abdeckung
(B) eine untere flüssigkeitsundurchlässige Schicht
(C) den zwischen Abdeckung (A) und Schicht (B) befindlichen wasserabsorbierenden Verbundstoff,
(D) wahlweise eine sich unmittelbar oberhalb und unterhalb des wasserabsorbierenden Verbundstoffs (C) sich befindende Tissueschicht und
(E) wahlweise eine zwischen Abdeckung (A) und wasserabsorbierenden Verbundstoff (C) sich befindende Aufnahme- und Verteilschicht.

Die Dicke des wasserabsorbierenden Verbundstoffs kann variiert werden. So kann der wasserabsorbierend Verbundstoff beispielsweise im hinteren Bereich weniger Material aufweisen. Aussparungen und Kanäle sind ebenfalls möglich.

Unter Hygieneartikel sind dabei beispielsweise Inkontinenzeinlagen und Inkontinenzhosen für Erwachsene oder Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 1 023 883 A2.

Die flüssigkeitsundurchlässige Schicht (B) besteht in der Regel aus einer Folie aus Polyethylen oder Polypropylen. Auf die Schicht (B) kann für besser Griffeigenschaften auf der Außenseite ein Flies auflaminiert sein.

Aufnahme- und Verteilschichten (E) werden typischerweise aus Vliesen hergestellt, die sehr gute Dochtwirkung haben, um die Flüssigkeit schnell aufzunehmen und zu verteilen. Sie verbessern auch das Rücknässen. Wenn durch Druck auf die Windel der wasserabsorbierende Verbundstoff Flüssigkeit abgibt, verhindert die Aufnahme- und Verteilschicht (E), dass diese Flüssigkeit mit der Haut des Nutzers in Berührung kommt.

Geeignete Vliese sind thermisch verbundene oder mit Harzen verklebte Fasern auf Basis von Polypropylen- und/oder Polyesterfasern mit einem Flächengewicht von 25 bis 70 gms, beispielsweise Curadis®, Curadis® EPS, Curadis® ATP und Curadis® RB (Albis SPA, IT).

Weitere geeignete Aufnahme- und Verteilschichten (E) werden durch "Airthroughbonding" erhalten und sind unter den Handelsmarken Acquitex® (Texus SPA, IT) und Dry Web® (Libeltex BVBA, NL) erhältlich.

Die gemäß dem erfindungsgemäßen Verfahren hergestellten wasserabsorbierenden Verbundstoffe weisen gegenüber den gemäß bisher üblichen Verfahren hergestellten Verbundstoffen eine verbesserte Geruchsinhibierung auf.

Die wasserabsorbierenden Polymerpartikel und Verbundstoffe werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Zentrifugenretentionskapazität (CRC, Pad)

Die Zentrifugenretentionskapazität (CRC) der wasserabsorbierende Verbundstoffe ("Pad") wird analog der von der EDANA empfohlenen Testmethode Nr. WSP 241.2-05 "Centrifuge Retention Capacity" mit folgenden Änderungen bestimmt:

- statt der wasserabsorbierenden Polymerpartikel (Superabsorber) wird ein ausgestanztes Pad (rund, 5 cm Durchmesser) pro Teebeutel, unabhängig vom Gewicht verwendet
- es wird ein Teebeutel (20 x 10) cm$^2$ verwendet (Punkt 6.1 der Methode)
- nur 5 Teebeutel pro Liter Salzlösung (Punkt 8.6 der Methode)
- das Pad wird vor dem Verschweißen des Teebeutels etwas auseinandergezogen
- es wird eine Fünffachbestimmung durchgeführt (Punkt 9.3 der Methode)

Zentrifugenretentionskapazität (CRC,)

Die Zentrifugenretentionskapazität (CRC) der wasserabsorbierenden Polymerpartikel wird gemäß der von der EDANA empfohlenen Testmethode Nr. WSP 241.2-05 "Centrifuge Retention Capacity" bestimmt.

Absorption unter einem Druck von 49,2 g/cm$^2$ (AUL0.7psi)

Die Absorption unter einem Druck von 49,2 g/cm$^2$ (AUL0.7psi) der wasserabsorbierenden Polymerpartikel wird analog der von der EDANA empfohlenen Testmethode Nr. WSP 242.2-05 "Absorption under Pressure" bestimmt, wobei statt eines Drucks von 21,0 g/cm$^2$ (AUL0.3psi) ein Druck von 49,2 g/cm$^2$ (AUL0.7psi) eingestellt wird.

Flüssigkeitsweiterleitung (SFC)

Die Flüssigkeitsweiterleitung (SFC) einer gequollenen Gelschicht unter Druckbelastung von 63,3 g/cm$^2$ (0,9 psi) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC [cm}^3\text{s/g] = (Fg(t=0)xL0)/(dxAxWP)},$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$.

Bakterieninduzierte Ammoniakfreisetzung

DSM1 Medium (Deutsche Sammlung von Mikroorganismen und Zellkulturen GmbH) wurde hergestellt aus 5,0 g/l Pepton aus Fleisch (Merck KGaA; DE; Art. Nr. 1.07214) und 3,0 g/l Fleischextrakt (Merck KGaA; DE; Art. Nr. 1103979) und auf pH = 7,0 eingestellt. 50 ml DSM1 Medium wurde mit Proteus mirabilis ATCC 14153 auf OD = 0,1 angeimpft und in einem 250 ml Erlenmeyerkolben mit Schikane für 15 Stunden bei 37°C und 220 Upm inkubiert. Die so hergestellten Kulturen hatten eine Zelldichte von ungefähr $10^9$ KBE/ml (OD = 2,0 - 2,5).

Der synthetische Urin wurde hergestellt aus 25 g/l Harnstoff (sterilfiltriert), 9,0 g/l Natriumchlorid, 1 g/l Pepton aus Fleisch und 1 g/l Fleischextrakt. Der synthetische Urin wurde vor Zugabe einer sterilfiltrierten konzentrierten Harnstofflösung autoklaviert.

Ein 125 ml Polypropylen-Histologiebecher wird autoklaviert und ein Pad (5 cm Durchmesser) hineingelegt. Die zur Aufnahme nötige Menge synthetischen Urins wird zugegeben (berechnet aus der Zentrifugenretentionskapazität). Der synthetische Urin wurde vorher mit Bakterienstammlösung entsprechend einer Gesamtkonzentration von ca. $10^6$ KBE/ml beimpft. Sofort nach Zugabe wird der mit einem Diffusionsteströhrchen vom Typ Dräger-Röhrchen® Ammoniak 20/a-D; Sach-Nr. 8101301 (Drägerwerk AG & Co. KGaA; DE;) versehene Deckel aufgeschraubt. Die Ammoniakentwicklung wurde bei 37°C über 48 Stunden beobachtet.

Beispiel

Cellulosefluff 1

Bögen aus Sulfatzellstoff NB416 (Weyerhaeuser Inc.; US) wurden in einem handelsüblichen Küchenmixer vom Typ KitchenAid® Ultra Power Standmixer bei maximaler Leistung aufgeschlossen.

Cellulosefluff 2

Cellulosefluff 1 wurde in einem handelsüblichen Küchenmixer vom Typ KitchenAid® Ultra Power Standmixer mit 0,5 Gew.-% Zinkperoxid (56gew.-%ig; Nitrochemie Aschau GmbH; DE) vorsichtig aber gleichmäßig vermischt und anschließend gleichmäßig mit 0,1 Gew.-% einer 50gew.-%igen wässrigen Lösung von Pluriol® E 400 besprüht.

Wasserabsorbierende Polymerpartikel 1

Es wurden handelsübliche wasserabsorbierende Polymerpartikel vom Typ HySorb® B7065 (BASF SE; DE) verwendet. HySorb® B7065 ist eine vernetzte, teilneutralisierte Polyacrylsäure mit einem Neutralisationsgrad von 75 mol-%. Die Polymerpartikel sind mit Denacol® EX410 oberflächennachvernetzt und weisen folgende Eigenschaften auf:

| | |
|---|---|
| CRC: | 30 g/g |
| AUL0.7psi: | 23 g/g |
| SFC: | $30 \times 10^{-7}$ |

Wasserabsorbierende Polymerpartikel 2

200g wasserabsorbierende Polymerpartikel 1 wurden mit 1,0 g Zinkperoxid (56gew.-%ig; Nitrochemie Aschau GmbH; DE) in eine Glasflasche eingewogen. Anschließend wurde diese Mischung in einen großen Porzellanmörser (16cm Innendurchmesser) überführt und dort für ca. 5 Minuten verrieben. Zusätzlich wurden die Proben nochmals 20 Minuten bei 46Upm im Taumelmischer homogenisiert. Im Anschluss wurde unter Rühren 0,28 g einer 50gew.-%igen wässrigen Lösung von Pluriol® E 400 aufgesprüht.

Herstellung der wasserabsorbierenden Verbundstoffe (allgemeine Herstellvorschrift)

3,0 g wasserabsorbierende Polymerpartikel werden zu sechs gleichen Portionen von 0,50 +/- 0,001 g auf Wägeschiffchen abgewogen.

4,5 g Cellulosefluff werden in sechs gleichen Portionen von 0,75 +/- 0,01 g geteilt.

Der wasserabsorbierende Verbundstoff wird wie folgt hergestellt:

Auf ein rechteckiges Drahtnetz mit einer Lange von 17,5 cm und einer Breite von 11 cm wird ein Tissue (SCA Hygiene Products AB; SE) aufgelegt, wobei das Tissue etwas über das Drahtnetz hinausragt. Oberhalb des Drahtnetzes befindet sich ein senkrechter Schacht gleiche Dimensionierung. In diesen Schacht, ca. 75 cm oberhalb des Drahtnetzes, rotiert eine längs eingebaute Bürste. Die Bürste hat eine Länge von 17,5 cm und einen Durchmesser von 10 cm. Die Bürste rotiert mit 13,5 Umdrehungen pro Sekunde. Unterhalb des Drahtnetzes mit dem Tissue wird Vakuum angelegt.

Es wird die erste Portion Cellulosefluff von oben auf die rotierende Bürste aufgegeben. Nach 25 Sekunden werden jeweils die erste Portion Polymer von oben auf die rotierende Bürste dosiert. Die Dosierungen von Cellulosefluff und wasserabsorbierenden Polymerpartikeln werden nach jeweils 25 Sekunden insgesamt noch zweimal wiederholt. Anschließend wird das Drahtnetz mit dem Tissue horizontal um 180° gedreht.

Nun werden die Dosierungen von Cellulosefluff und wasserabsorbierenden Polymerpartikeln insgesamt noch dreimal wiederholt, der entstandene wasserabsorbierenden Verbundstoff von Hand mit einem Stempel mit einer Lange von 15 cm und einer Breite von 8,5 cm festgedrückt, von dem Tissue abgenommen und in ein Tissue (SCA Hygiene Products AB; SE) mit einer Länge von 37 cm und einer Breite von 24 cm eingeschlagen.

Verbundstoff 1

Gemäß der allgemeinen Herstellvorschrift wurde aus Cellulosefluff 2 und wasserabsorbierenden Polymerpartikeln 1 ein wasserabsorbierender Verbundstoff hergestellt.

Verbundstoff 2

Gemäß der allgemeinen Herstellvorschrift wurde aus Cellulosefluff 1 und wasserabsorbierenden Polymerpartikeln 2 ein wasserabsorbierender Verbundstoff hergestellt.

Verbundstoff 3

Gemäß der allgemeinen Herstellvorschrift wurde aus Cellulosefluff 1 und wasserabsorbierenden Polymerpartikeln 1 ein wasserabsorbierender Verbundstoff hergestellt, wobei nach der Drehung des Drahtneztes um 180° 15mg Zinkperoxid (56gew.-%ig; Nitrochemie Aschau GmbH; DE) gleichmäßig aufgebracht wurden.

Herstellung der Windeln

Je eine Windel vom Typ Pampers Simply Dry, Größe 4, wurde vorsichtig geöffnet, der absorbierende Kern komplett entfernt und jeweils durch einen der hergestellten wasserabsorbierenden Verbundstoffe 1 bis 3 ersetzt. Der wasserabsorbierende Verbundstoff wurde jeweils mittig in der Windel platziert.

Aus der Mitte der Windel wurde ein Pad mit 5 cm Durchmesser ausgestanzt und anschließend mit dem Pad die bakterieninduzierte Ammoniakfreisetzung bestimmt.

Tab. 1: baterieninduzierte Ammoniakfreisetzung

|  | Zeit bis zu einer Ammniakfreisetzung von 1500 ppm/h |
|---|---|
| Verbundstoff 1 | nach 48 h noch kein Ammoniak nachweisbar |
| Verbundstoff 2*) | 32,25h |
| Verbundstoff 3*) | 30,75h |
| *) nicht erfindungsgemäß | |

**Patentansprüche**

1. Verfahren zur Herstellung geruchsinhibierender wasserabsorbierender Verbundstoffe, enthaltend wasserabsorbierende Polymerpartikel und Fasern, **dadurch gekennzeichnet, dass** a) die Fasern mit mindestens einem Metallperoxid, Metallhyperoxid oder Metallozonid vorgemischt werden und danach b) die wasserabsorbierenden Polymerpartikel mit der Vormischung a) gemischt werden.

2. Verfahren gemäß Anspruch 1, **dadurch gekennzeichnet, dass** das Metallperoxid das Peroxid eines Metalls der 1. Hauptgruppe, der 2. Hauptgruppe und/oder der 2. Nebengruppe des Periodensystems der Elemente ist.

3. Verfahren gemäß Anspruch 1 oder 2, **dadurch gekennzeichnet, dass** das Metallperoxid das Peroxid eines Metalls der 2. Nebengruppe des Periodensystems der Elemente ist.

4. Verfahren gemäß einem der Ansprüche 1 bis 3, **dadurch gekennzeichnet, dass** das Metallperoxid Zinkperoxid ist.

5. Verfahren gemäß einem der Ansprüche 1 bis 4, **dadurch gekennzeichnet, dass** der Verbundstoff von 0,001 bis 5 Gew.-% des Metallperoxids, Metallhyperoxids oder Metallozonids enthält.

6. Verfahren gemäß einem der Ansprüche 1 bis 5, **dadurch gekennzeichnet, dass** die Fasern Zellulosefasern sind.

7. Verfahren gemäß einem der Ansprüche 1 bis 6, **dadurch gekennzeichnet, dass** der Verbundstoff höchstens 70 Gew.-% Fasern enthält.

8. Verfahren gemäß einem der Ansprüche 1 bis 7, **dadurch gekennzeichnet, dass** die wasserabsorbierenden Polymerpartikel zu mindestens 50 Gew.-% polymerisierte Acrylsäure und/oder deren Salze enthalten.

9. Verfahren gemäß einem der Ansprüche 1 bis 8, **dadurch gekennzeichnet, dass** die wasserabsorbierenden Polymerpartikel eine Zentrifugenretentionskapazität von mindestens 15 g/g aufweisen.

10. Verbundstoff, erhältlich gemäß einen der Ansprüche 1 bis 9

11. Hygieneartikel, enthaltend einen gemäß einem der Ansprüche 1 bis 9 hergestellten Verbundstoff.


**Claims**

1. A process for producing odor-inhibiting water-absorbing composites comprising water-absorbing polymer particles and fibers, which comprises a) premixing the fibers with at least one metal peroxide, metal hyperoxide or metal ozonide and then b) mixing the water-absorbing polymer particles with the premix a).

2. The process according to claim 1, wherein the metal peroxide is the peroxide of a metal of main group 1, of main group 2 and/or of transition group 2 of the Periodic Table of the Elements.

3. The process according to claim 1 or 2, wherein the metal peroxide is the peroxide of a metal of transition group 2 of the Periodic Table of the Elements.

4. The process according to any of claims 1 to 3, wherein the metal peroxide is zinc peroxide.

5. The process according to any of claims 1 to 4, wherein the composite comprises from 0.001 to 5% by weight of the metal peroxide, metal hyperoxide or metal ozonide.

6. The process according to any of claims 1 to 5, wherein the fibers are cellulose fibers.

7. The process according to any of claims 1 to 6, wherein the composite comprises at most 70% by weight of fibers.

8. The process according to any of claims 1 to 7, wherein the water-absorbing polymer particles consist to an extent of at least 50% by weight of polymerized acrylic acid and/or salts thereof.

9. The process according to any of claims 1 to 8, wherein the water-absorbing polymer particles have a centrifuge retention capacity of at least 15 g/g.

10. A composite obtainable according to any of claims 1 to 9.

11. A hygiene article comprising a composite produced according to any of claims 1 to 9.


**Revendications**

1. Procédé pour la production de matériaux composites absorbant l'eau, inhibant les odeurs, contenant des particules polymères absorbant l'eau et des fibres, **caractérisé en ce que** a) les fibres sont prémélangées avec au moins un peroxyde de métal, un hyperoxyde de métal ou un ozonure de métal et ensuite b) les particules polymères absorbant l'eau sont mélangées avec le prémélange a).

2. Procédé selon la revendication 1, **caractérisé en ce que** le peroxyde de métal est le peroxyde d'un métal du 1er groupe principal, du 2ème groupe principal et/ou du 2ème groupe secondaire du système périodique des éléments.

3. Procédé selon la revendication 1 ou 2, **caractérisé en ce que** le peroxyde de métal est le peroxyde d'un métal du 2ème groupe secondaire du système périodique des éléments.

4. Procédé selon l'une quelconque des revendications 1 à 3, **caractérisé en ce que** le peroxyde de métal est le peroxyde de zinc.

5. Procédé selon l'une quelconque des revendications 1 à 4, **caractérisé en ce que** le matériau composite contient 0,001 à 5% en poids du peroxyde de métal, de l'hyperoxyde de métal ou de l'ozonure de métal.

6. Procédé selon l'une quelconque des revendications 1 à 5, **caractérisé en ce que** les fibres sont des fibres de cellulose.

7. Procédé selon l'une quelconque des revendications 1 à 6, **caractérisé en ce que** le matériau composite contient au plus 70% en poids de fibres.

8. Procédé selon l'une quelconque des revendications 1 à 7, **caractérisé en ce que** les particules polymères absorbant l'eau contiennent à raison d'au moins 50% en poids d'acide acrylique polymérisé et/ou ses sels.

9. Procédé selon l'une quelconque des revendications 1 à 8, **caractérisé en ce que** les particules polymères absorbant l'eau présentent une capacité de rétention dans une centrifugeuse d'au moins 15 g/g.

10. Matériau composite, pouvant être obtenu selon l'une quelconque des revendications 1 à 9.

11. Articles hygiéniques contenant un matériau composite produit selon l'une quelconque des revendications 1 à 9.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 627512 A
- GB 2377890 A
- JP 2001039802 A
- JP 2001115042 A
- US 6649805 B
- WO 2010096595 A
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C
- DE 19854573 A1
- DE 19854574 A1
- DE 10204937 A1
- DE 10334584 A1
- EP 1199327 A2
- WO 2003031482 A1
- DE 3713601 A1
- WO 9526209 A1
- US 3556932 A
- WO 9111162 A1
- US 3224926 A
- US 3440135 A
- US 3932209 A
- US 4035147 A
- US 4822453 A
- US 4888093 A
- US 4898642 A
- US 5137537 A
- WO 9957355 A1
- EP 1023883 A2
- EP 0640330 A1

**In der Beschreibung aufgeführte Nicht-Patentliteratur**

- Die Herstellung von Hygieneartikeln wird in der Monographie. **F.L. BUCHHOLZ ; A.T. GRAHAM.** Modern Superabsorbent Polymer Technology. Wiley-VCH, 1998, 252-258
- **F.L. BUCHHOLZ ; A.T. GRAHAM.** Modern Superabsorbent Polymer Technology. Wiley-VCH, 1998, 71-103